# EUROPEAN PATENT SPECIFICATION

(11) **EP 1 438 925 B1**
(45) Date of publication and mention of the grant of the patent: **28.02.2007**
(21) Application number: 02028932.8
(22) Date of filing: 23.12.2002
(51) Int. Cl.: A61C 1/00, A61L 2/18, A61L 2/24

(54) **A method for sanitising the water circuits of dental units and a dental unit implementing the method**
Verfahren zur Sanierung von Wasserleitungen zahnärtztlicher Einheiten und zahnärtztliche Einheit zur Durchführung dieses Verfahrens
Assainissement de conduits d'eau dans les unités dentaires et unité dentaire mettant en oeuvre cette méthode

(43) Date of publication of application: 21.07.2004
(73) Proprietor: CASTELLINI S.p.A., I-40013 Castel Maggiore (Bologna) (IT)
(72) Inventor: Castellini, Franco, 40124 Bologna (IT)
(74) Representative: Lanzoni, Luciano

(56) References cited:
- EP-A- 1 344 499
- WO-A-00/33757
- WO-A-01/95948
- WO-A-02/15811
- WO-A-96/29098
- US-A1- 2002 112 743

## Description

The present invention relates to a method for sanitising the water circuits of dental units and to a dental unit implementing the method.

In the manufacturing of dental equipment, one of the fundamental parts, if not the "heart", of a dental unit is its water and air circuit, where the water line supplies fluids used by dental equipment and patients (water or physiological saline for tumblers and handpieces), or consumer units (swilling water for the spittoon), while the air line is used for certain items of equipment (air spray handpieces, cooling air and driving air).

With increases in general standards of hygiene and with dental apparatus and equipment becoming more and more "fragile", several design solutions have been found for the water and air circuits of dental units, not only to guarantee their efficient operation and durability but also to maintain the sterility of the conduits during patient treatments. Considering that the basic structure of these fluid circuits comprises a first main line supplying water from the mains, and a second main line supplying air from an external source (compressor), each of which has a plurality of branches leading to the operating and accessory equipment of the dental unit, different systems have been designed on the basis of different methods aimed at improving the functioning and disinfection of these fluid lines or parts of them. In particular, the present specification focuses attention on the water line which is disinfected according to two different methods, one with a continuous cycle and the other with a discontinuous cycle, and both requiring additional devices to be fitted to the basic structure of the circuit.

Document EP A 1344499, filed on 12/03/2002 and published on 17/09/2003, discloses a method and a device for cleaning / disinfecting / sterilising the water circuits of dental units. The method disclosed by EP A 1344499 comprises the steps of introducing a cleaning / disinfecting / sterilising fluid into the water circuit in two or more sub-steps; holding the cleaning / disinfecting / sterilising fluid in the water circuit for a predetermined length of time; and discharging the cleaning / disinfecting / sterilising fluid out of the circuit through endpieces of supply branches.

Document WO 96/29098 discloses a method for disinfecting the water circuit of a dental unit, said method comprising the steps of introducing a disinfectant solution into the water circuit; holding said solution therein for a predetermined length of time; draining the solution out of the circuit by using a gas or evacuating it with vacuum; and introducing again a disinfectant solution.

The present specification is concerned in particular with the solutions based on the discontinuous disinfection/sterilisation cycle. In this type of cycle, as disclosed in patent publications EP - 111.249 and EP - 317.521 (the latter being by the present Applicant), the mains water supply is shut off, and a dedicated branch equipped with an independent tank is used to feed sterilising liquid into the conduits that supply water to the handpieces.

After a preset time, depending on the quality of disinfection/sterilisation required and the properties of the sterilising liquid, the line is opened again and the sterilising liquid drained out.

The drainage of the sterilising liquid is performed by flushing water (or a user fluid) supplied by the main line (or by a dedicated line) and opening the control valves on the handpieces so that the water or user fluid expels the sterilising liquid, rinses the water line and flows out into an appropriate drain.

Although this method, which has been used on dental units for some time, has proved to be very effective and practical, the Applicant, in line with a policy of continual improvement of dental unit sanitising procedures, has continued to conduct numerous studies and research projects which have revealed that the sanitising product, if supplied in a single, predetermined dose and held in the circuit for the entire contact period, tends to deteriorate, thus locally reducing the effect of the sanitising treatment on account of a loss of effective product concentration in certain parts of the system.

The aim of the present invention is to overcome the above mentioned disadvantages by providing a method for sterilising / disinfecting the water circuit of a dental unit such as to optimise the sanitising action of the disinfectant / sterilising liquid by keeping the liquid at a constantly high level of effectiveness at all times and in all parts of the circuit.

The above mentioned aim is achieved in a method for sanitising water circuits of dental units comprising dental handpieces and a water circuit for supplying fluid from a main supply through corresponding circuit legs or branches, the method comprising at least the following steps: introducing the disinfectant / sterilising liquid in the water circuit for a predetermined length of time and draining the disinfectant / sterilising liquid out of the circuit through the circuit legs or branches; the step of introducing the disinfectant / sterilising liquid being divided into two or more sub-steps, each consisting of an inflow of a predetermined quantity or dose of the disinfectant / sterilising liquid, alternated with steps of waiting for the next inflow.

The technical characteristics of the invention, with reference to the above aims, are clearly described in the claims below and its advantages are apparent from the detailed description which follows, with reference to the accompanying drawings which illustrate a preferred embodiment of the invention provided merely by way of example without restricting the scope of the inventive concept, and in which:
- Figure 1 shows a diagram representing the water circuit of a dental unit that implements the method according to the present invention for sanitising the water circuit itself;
- Figure 2 shows a graph representing the effects obtained with the method according to the present invention.

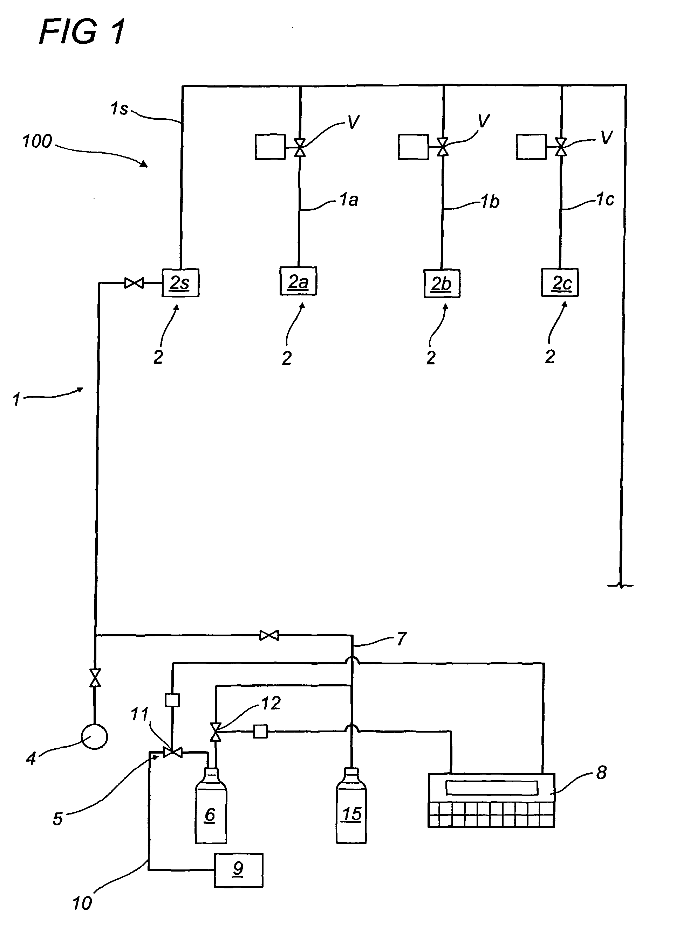

With reference to the accompanying drawings, in particular Figure 1, the method according to the invention is used to sanitise the water circuit 1 of a dental unit 100.

The dental unit 100 is of the type comprising, at least insofar as is relevant to the present invention, medical instruments such as dental handpieces 2 (represented as a block in the drawings) equipped with shutoff valves V, and a water circuit 1 for feeding a fluid from a main supply 4 (water) or from an independent circuit leg that supplies sterile liquid through a suitable container 15.

The handpieces 2 (which may be, for example, a micromotor 2a, a turbine 2b, an ablator 2c and a syringe 2s) are supplied through corresponding legs or branches 1a, 1b, 1c, 1s of the circuit 1, which are equipped with the valves V.

The method according to the invention comprises at least the following steps:
- introducing a disinfectant / sterilising liquid in the water circuit 1 for a predetermined length of time;
- draining the disinfectant / sterilising liquid out of the circuit 1 through the circuit legs or branches 1a, 1b, 1c, 1s.

Obviously and as is well within the knowledge of experts in the trade familiar with methods of this kind, the valves V are opened and closed during these steps in order to allow the fluids to flow into the water circuit 1, be held there and then drained out.

In this particular case, the step of introducing the disinfectant / sterilising liquid is divided into two or more sub-steps, each consisting of an inflow of a predetermined quantity or dose of the disinfectant / sterilising liquid, alternated with steps of waiting for the next inflow of the liquid. In other words, each of these sub-steps of disinfectant / sterilising liquid inflow is such that the predetermined quantity or dose of the liquid is sufficient to renew the liquid (by completely filling) the water circuit 1, while the waiting time periods between one inflow and the next depends on the time the sanitising product remains active before it starts losing its sanitising charge. Thus, the total contact time, consisting of the time taken for the inflow steps added to the time periods of the steps of waiting between one inflow and another, is a function of the optimum time periods to sterilise / sanitise the circuit 1 and its branches.

Evidently, when a new dose of disinfectant / sterilising liquid flows in, the dose already inside the water circuit 1 is flushed out of the circuit branches 1a, 1b, 1c, 1s.

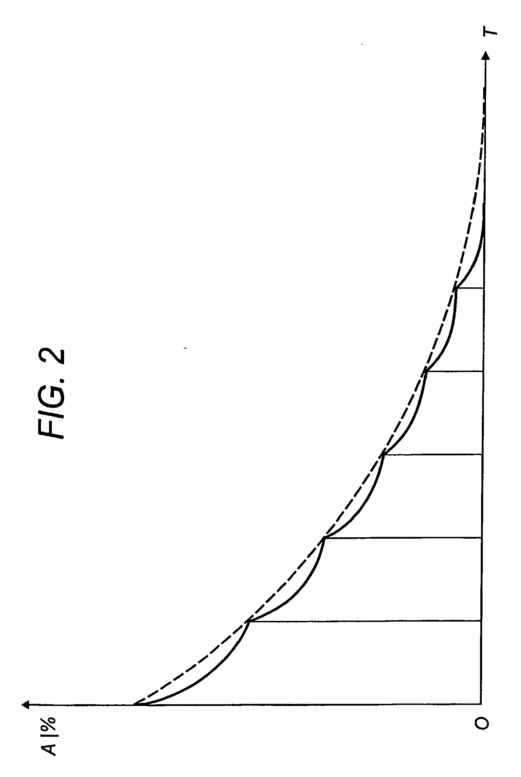

The sanitising effect of the successive inflows of disinfectant / sterilising liquid is illustrated purely by way of example in the graph of Figure 2, where the x-axis represents the time T and the y-axis the percentage of polluting agents AI present in the water circuit. A comparison of the sanitising performance of a traditional cycle (represented by the dashed line) with that of the new cycle, divided into sub-steps, according to the present invention (represented by the continuous line) shows the latter's greater effectiveness in destroying the polluting agents per unit time, with obvious advantages in terms of overall water circuit hygiene.

The aforementioned sub-steps of feeding a certain quantity of disinfectant / sterilising liquid are performed by feed means 5 acting on an independent container 6 of the disinfectant / sterilising liquid connected to the water circuit 1 through a first independent circuit branch 7.

In the embodiment illustrated, the dental unit 100 may be equipped with a microprocessor unit 8 that controls the main and accessory functions of the dental unit 100.

The microprocessor unit 8 can be advantageously used to program the feed means 5 so that the disinfectant / sterilising liquid is fed into the circuit in a predetermined, optimum dose during each inflow sub-step and left to take action for an optimum length of waiting time before the next dose is fed in.

The method according to the invention may comprise a further step of heating the disinfectant / sterilising liquid just before it is fed into the water circuit 1: this is to further improve and speed up the biocidal action of the disinfectant / sterilising liquid.

The dental unit that implements the method described above therefore comprises the above mentioned means 5 for the controlled infeed of the disinfectant / sterilising liquid acting on a container 6 in which the liquid is stored and designed to supply the water circuit 1 with disinfectant / sterilising liquid in a plurality of predetermined doses in such a way as to provide an active sanitising action.

As mentioned above, the dental unit 100 comprises a microprocessor unit 8 that controls the main and accessory functions of the dental unit 100, the microprocessor unit 8 controlling and activating the feed means 5 in such a way as to alternate the steps of feeding the plurality of doses of disinfectant / sterilising liquid into the water circuit 1 with steps of stopping the flow of the liquid.

The dental unit 100 further comprises an air circuit 9 for supplying air used by the dental unit 100 in a well-known manner for some of its main and accessory functions. The air circuit 9 is illustrated as a block and has branches connected with the parts relevant to the present invention purely by way of non-restrictive example.

The feed means 5 comprise a branch 10 of the air circuit 9. The circuit branch 10 is located in the container 6 of the disinfectant / sterilising liquid and is activated by a shutoff element 11 (for example a valve) controlled by the microprocessor unit 8 so as to allow the doses of the disinfectant / sterilising liquid to flow into the first independent branch 7.

The first independent branch 7 is equipped with a valve element 12 designed to stop and start the flow of disinfectant / sterilising liquid from the container 6 and, by activating the shutoff element 11, the flow of air through the circuit branch 10. In practice, the valve element 12 and the shutoff element 11 are switched on and off in synchrony with each other by the microprocessor unit 8 in order to obtain the correct liquid inflow and hold steps according to the programmed doses and time intervals.

The method and dental unit as described above therefore achieve the preset aims by allowing inflows and outflows of successive doses of disinfectant / sterilising liquid to be specifically programmed in such a way as to destroy polluting agents quickly and at a constant rate without necessitating more time than traditional cycles to accomplish a complete cycle of sure sterilisation / disinfection.

## Claims

1. A method for sanitising water circuits (1) of dental units comprising medical instruments, such as dental handpieces (2), and a water circuit (1) for supplying liquid from a main supply (4) through corresponding circuit legs or branches (1a, 1b, 1c, 1s); the method comprising at least the following steps: introducing the disinfectant / sterilising liquid in the water circuit (1) for a predetermined length of time and draining the disinfectant / sterilising liquid out of the circuit (1) through the circuit legs or branches (1a, 1b, 1c, 1s), the method being wherein the step of introducing the disinfectant / sterilising liquid is divided into two or more sub-steps, each consisting of an inflow of a predetermined quantity or dose of the disinfectant / sterilising liquid, alternated with steps of waiting for the next inflow.

2. The method according to claim 1, **characterised in that** the quantity or dose of disinfectant / sterilising liquid flowing in during each of the sub-steps is predetermined and sufficient to completely fill the water circuit (1) and renew the liquid in it, the waiting time periods between one inflow and the next being such that the disinfectant / sterilising liquid in the water circuit (1) is maintained at an effective concentration.

3. The method according to claim 1, **characterised in that** the sub-steps of feeding a certain quantity of disinfectant / sterilising liquid are performed by feed means (5) acting on an independent container (6) of the disinfectant / sterilising liquid connected to the water circuit (1) through a first independent circuit branch (7).

4. The method according to claim 1, where the dental unit (100) is equipped with a microprocessor unit (8) that controls the main and accessory functions of the dental unit (100), the method being **characterised in that** the sub-steps of feeding in the doses of disinfectant / sterilising liquid are performed by feed means (5) controlled by the microprocessor unit (8) so that the disinfectant / sterilising liquid is fed into the circuit for a predetermined inflow time and left to take action in the circuit for a predetermined length of waiting time before the next dose is fed in.

5. The method according to claim 1, **characterised in that** the step of introducing the disinfectant / sterilising liquid comprises a step of heating the disinfectant / sterilising liquid flowing into the water circuit (1).

6. The method according to claim 1, **characterised in that** each sub-step of feeding a dose of disinfectant / sterilising liquid is performed at the same time as a step of draining out the dose previously fed into the water circuit (1).

7. A dental unit comprising at least one main water circuit (1) for supplying a liquid to each handpiece (2) or accessory item of dental equipment present on the dental unit (100), and at least one independent circuit branch (7) connected to the water circuit (1) and supplying the water circuit (1) with at least one disinfectant / sterilising liquid from a container (6), the dental unit (100) being **characterised in that** it comprises means (5) for the controlled infeed of the disinfectant / sterilising liquid acting on the container (6) and designed to supply the water circuit (1) with disinfectant / sterilising liquid in a plurality of predetermined successive doses in such a way as to enable each dose of disinfectant / sterilising liquid to remain in the circuit (1) for as long as it remains active.

8. The dental unit according to claim 7, where the dental unit (100) comprises a microprocessor unit (8) to control the main and accessory of the dental unit (100), **characterised in that** the disinfectant / sterilising liquid feed means (5) are controlled by the microprocessor unit (8) in such a way as to alternate the steps of feeding the plurality of doses of disinfectant / sterilising liquid into the water circuit (1) with steps of stopping the flow of the liquid.

9. The dental unit according to claims 7 and 8, where the dental unit (100) has an air supply circuit (9) used for its main and accessory functions, the dental unit being **characterised in that** the feed means (5) comprise a branch (10) of the air circuit (9) that is located in the container (6) of the disinfectant / sterilising liquid and activated by a shutoff element (11) controlled by the microprocessor unit (8) so as to allow the doses of the disinfectant / sterilising liquid to flow into the first independent branch (7).

10. The dental unit according to claim 9, **characterised in that** the first independent branch (7) is equipped with valve means (12) designed to stop and start the flow of disinfectant / sterilising liquid from the container (6) and, by activating the shutoff element (11), the flow of air through the air circuit branch (10).

11. The dental unit according to claim 10, **characterised in that** the valve means (12) and the shutoff element (11) can be switched on and off by the microprocessor unit (8).

## Patentansprüche

1. Ein Verfahren zur Sanierung von Wasserleitungen (1) zahnärztlicher Einheiten, die medizinische Instrumente beinhalten, wie zahnärztliche Handstücke (2) und eine Wasserleitung (1) für die Zufuhr von Flüssigkeit von einer Hauptversorgung (4) über entsprechende Leitungszweige oder Abzweigungen (1a, 1b, 1c, 1s); wobei das Verfahren zumindest folgende Schritte umfasst: Einleiten der Desinfektions- / Sterilisierflüssigkeit in die Wasserleitung (1) für eine vorbestimmte Zeitdauer und Ablassen der Desinfektions- / Sterilisierflüssigkeit aus dem Leitungskreislauf (1) über die Leitungszweige oder Abzweigungen (1a, 1b, 1c, 1s), wobei der Schritt des Einleitens der Desinfektions- / Sterilisierflüssigkeit in zwei oder mehr Unterschritte unterteilt ist, jeweils bestehend aus einem Zufluss einer vorbestimmten Menge oder Dosis Desinfektions- / Sterilisierflüssigkeit, abwechselnd mit Schritten des Wartens auf den nächsten Zufluss.

2. Verfahren nach Anspruch 1, **dadurch gekennzeichnet, dass** die Menge oder Dosis Desinfektions- / Sterilisierflüssigkeit, die während jedes Unterschrittes zufließt, vorbestimmt und ausreichend ist, um die Wasserleitung (1) vollständig zu füllen und die darin enthaltene Flüssigkeit zu erneuern, und dass die Wartezeiten zwischen zwei aufeinanderfolgenden Zuflüssen so gewählt sind, dass die Desinfektions- / Sterilisierflüssigkeit in der Wasserleitung (1) auf einer wirksamen Konzentration gehalten wird.

3. Verfahren nach Anspruch 1, **dadurch gekennzeichnet, dass** die Unterschritte des Zuführens einer bestimmten Menge Desinfektions- / Sterilisierflüssigkeit durch Zufuhrmittel (5) ausgeführt werden, die auf einen unabhängigen Behälter (6) mit Desinfektions- / Sterilisierflüssigkeit einwirken, der über eine erste unabhängige Leitungsabzweigung (7) mit der Wasserleitung (1) verbunden ist.

4. Verfahren nach Anspruch 1, worin die zahnärztliche Einheit (100) mit einer Mikroprozessor-Einheit (8) ausgestattet ist, welche die Haupt- und Nebenfunktionen der zahnärztlichen Einheit (100) steuert, wobei das Verfahren **dadurch gekennzeichnet ist, dass** die Unterschritte des Zuführens der Dosen Desinfektions- / Sterilisierflüssigkeit durch Zufuhrmittel (5) ausgeführt werden, die von der Mikroprozessor-Einheit (8) so gesteuert werden, dass die Desinfektions- / Sterilisierflüssigkeit für eine vorbestimmte Zuflussdauer in die Leitung eingespeist wird und dann im Leitungskreislauf für eine Wartezeit vorbestimmter Dauer einwirken kann, bevor die nächste Dosis zugeführt wird.

5. Verfahren nach Anspruch 1, **dadurch gekennzeichnet, dass** der Schritt des Einleitens der Desinfektions- / Sterilisierflüssigkeit einen Schritt zum Aufheizen der in die Wasserleitung (1) einfließenden Desinfektions- / Sterilisierflüssigkeit beinhaltet.

6. Verfahren nach Anspruch 1, **dadurch gekennzeichnet, dass** jeder Unterschritt des Zuführens einer Dosis Desinfektions- / Sterilisierflüssigkeit gleichzeitig mit einem Schritt des Ablassens der zuvor in die Wasserleitung (1) eingespeisten Dosis ausgeführt wird.

7. Zahnärztliche Einheit, bestehend aus mindestens einer Hauptwasserleitung (1) für die Zufuhr einer Flüssigkeit zu jedem Handstück (2) oder Zubehörteil der zahnärztlichen Ausrüstung auf einer zahnärztlichen Einheit (100), und mindestens einer unabhängigen Leitungsabzweigung (7), die mit der Wasserleitung (1) verbunden ist und die Wasserleitung (1) mit zumindest einer Desinfektions- / Sterilisierflüssigkeit aus einem Behälter (6) versorgt, wobei die zahnärztliche Einheit (100) **dadurch gekennzeichnet ist, dass** sie Mittel (5) für die gesteuerte Zufuhr der Desinfektions- / Sterilisierflüssigkeit umfasst, die auf den Behälter (6) einwirken und dafür konzipiert sind, die Wasserleitung (1) in mehreren, vorbestimmten, aufeinanderfolgenden Dosierungen so mit Desinfektions- / Sterilisierflüssigkeit zu versorgen, dass jede Dosis Desinfektions- / Sterilisierflüssigkeit so lange in der Leitung (1) verbleiben kann, wie sie wirksam bleibt.

8. Zahnärztliche Einheit nach Anspruch 7, worin die zahnärztliche Einheit (100) eine Mikroprozessor-Einheit (8) zur Steuerung der Haupt- und Nebenfunktionen der zahnärztlichen Einheit (100) beinhaltet, **dadurch gekennzeichnet, dass** die Zufuhrmittel (5) für die Desinfektions- / Sterilisierflüssigkeit von der Mikroprozessor-Einheit (8) so gesteuert werden, dass sich die Schritte des Zuführens mehrerer Dosen der Desinfektions- / Sterilisierflüssigkeit in die Wasserleitung (1) mit Schritten abwechseln, in denen der Flüssigkeitszufluss gestoppt wird.

9. Zahnärztliche Einheit nach den Ansprüchen 7 und 8, worin die zahnärztliche Einheit (100) eine Luftzufuhrleitung (9) aufweist, die für ihre Haupt- und Nebenfunktionen verwendet wird, und wobei die zahnärztliche Einheit **dadurch gekennzeichnet ist, dass** die Zufuhrmittel (5) eine Abzweigung (10) der Luftleitung (9) umfassen, die in dem Behälter (6) der Desinfektions- / Sterilisierflüssigkeit angeordnet ist und durch ein von der Mikroprozessor-Einheit (8) gesteuertes Absperrelement (11) so betätigt wird, dass das Einfließen der Dosen Desinfektions- / Sterilisierflüssigkeit in die erste unabhängige Abzweigung (7) ermöglicht wird.

10. Zahnärztliche Einheit nach Anspruch 9, **dadurch gekennzeichnet, dass** die erste unabhängige Abzweigung (7) mit Ventilelementen (12) ausgerüstet ist, die dafür konzipiert sind, den Fluss der Desinfektions- / Sterilisierflüssigkeit von dem Behälter (6) sowie, durch Betätigen des Absperrelements (11), den Luftdurchfluss durch die Luftleitungsabzweigung (10) zu stoppen und zu starten.

11. Zahnärztliche Einheit nach Anspruch 10, **dadurch gekennzeichnet, dass** die Ventilelemente (12) und das Absperrelement (11) durch die Mikroprozessor-Einheit (8) ein- und ausgeschaltet werden können.

## Revendications

1. Une méthode pour aseptiser des circuits d'eau (1) d'unités dentaires comprenant des instruments médicaux, tels que des pièces à main dentaires (2), et un circuit d'eau (1) destiné à alimenter un liquide provenant d'une alimentation principale (4) à travers des branches ou ramifications de circuit (1a, 1b, 1c, 1s) correspondantes ; telle méthode comprenant au moins les phases suivantes : introduction du liquide de désinfection / stérilisation dans le circuit d'eau (1) pendant un intervalle de temps prédéfini et évacuation du liquide de désinfection / stérilisation hors du circuit (1) à travers les branches ou ramifications de circuit (1a, 1b, 1c, 1s), ladite méthode étant **caractérisée en ce que** la phase d'introduction du liquide de désinfection / stérilisation est subdivisée en deux sous-phases ou plus, consistant chacune en un afflux d'une quantité ou dose prédéfinie de liquide de désinfection / stérilisation, alternées avec des phases d'attente de l'afflux suivant.

2. La méthode selon la revendication 1, **caractérisée en ce que** la quantité ou dose de liquide de désinfection / stérilisation affluant lors de chacune des sous-phases est prédéfinie et suffisante pour remplir complètement le circuit d'eau (1) et renouveler le liquide qu'il contient, les intervalles de temps d'attente entre un afflux et le suivant étant tels que le liquide de désinfection / stérilisation dans le circuit d'eau (1) est maintenu à une concentration efficace.

3. La méthode selon la revendication 1, **caractérisée en ce que** les sous-phases d'afflux d'une quantité donnée de liquide de désinfection / stérilisation sont effectuées par des moyens d'alimentation (5) agissant sur un récipient indépendant (6) de liquide de désinfection / stérilisation relié au circuit d'eau (1) par l'intermédiaire d'une première branche de circuit (7) indépendante.

4. La méthode selon la revendication 1, où l'unité dentaire (100) est équipée d'une unité à microprocesseur (8) qui contrôle les fonctions principales et accessoires de l'unité dentaire (100) elle-même, la méthode étant **caractérisée en ce que** les sous-phases d'afflux des doses de liquide de désinfection / stérilisation sont effectuées par des moyens d'alimentation (5) asservis à l'unité à microprocesseur (8) de manière à ce que le liquide de désinfection / stérilisation afflue dans le circuit pendant des temps d'afflux prédéfinis et agisse dans ce même circuit pendant un intervalle prédéfini de temps d'attente avant l'afflux de la dose suivante.

5. La méthode selon la revendication 1, **caractérisée en ce que** la phase d'introduction du liquide de désinfection / stérilisation comprend une phase de chauffage du liquide de désinfection / stérilisation affluant dans le circuit d'eau (1).

6. La méthode selon la revendication 1, **caractérisée en ce que** chaque sous-phase d'afflux d'une dose de liquide de désinfection / stérilisation est effectuée en même temps qu'une phase d'évacuation de la dose précédemment alimentée dans le circuit d'eau (1).

7. Une unité dentaire comprenant au moins un circuit d'eau (1) principal destiné à alimenter un liquide à chaque pièce à main (2) ou élément d'équipement dentaire accessoire présent sur l'unité dentaire (100), et au moins une branche de circuit (7) indépendante reliée au circuit d'eau (1) et alimentant ce même circuit d'eau (1) avec au moins un liquide de désinfection / stérilisation provenant d'un récipient (6), l'unité dentaire (100) étant **caractérisée en ce qu'**elle comprend des moyens (5) pour l'alimentation contrôlée du liquide de désinfection / stérilisation qui agissent sur le récipient (6) et sont destinés à alimenter le circuit d'eau (1) avec un liquide de désinfection / stérilisation selon une pluralité de doses successives prédéfinies de manière à permettre à chaque dose de liquide de désinfection / stérilisation de rester dans le circuit (1) aussi longtemps que le liquide lui-même est actif.

8. L'unité dentaire selon la revendication 7, où l'unité dentaire (100) comprend une unité à microprocesseur (8) destinée à contrôler les fonctions principales et accessoires de cette même unité dentaire (100), **caractérisée en ce que** les moyens (5) d'alimentation du liquide de désinfection / stérilisation sont asservis à l'unité à microprocesseur (8) de manière à alterner les phases d'afflux de la pluralité de doses de liquide de désinfection / stérilisation dans le circuit d'eau (1) avec des phases de coupure du flux de liquide.

9. L'unité dentaire selon les revendications 7 et 8, où l'unité dentaire (100) comprend un circuit d'alimentation en air (9) utilisé pour ses fonctions principales et accessoires, l'unité dentaire étant **caractérisée en ce que** les moyens d'alimentation (5) comprennent une branche (10) du circuit d'air (9) qui est située dans le récipient (6) de liquide de désinfection / stérilisation et est activée par un élément d'arrêt (11) asservi à l'unité à microprocesseur (8) de manière à permettre aux doses de liquide de désinfection / stérilisation d'affluer dans la première branche indépendante (7).

10. L'unité dentaire selon la revendication 9, **caractérisée en ce que** la première branche indépendante (7) est équipée de moyens à vanne (12) destinés à arrêter ou à activer le flux de liquide de désinfection / stérilisation provenant du récipient (6) et, par le biais de l'activation de l'élément d'arrêt (11), le flux d'air à travers la branche (10) du circuit d'air.

11. L'unité dentaire selon la revendication 10, **caractérisée en ce que** les moyens à vanne (12) et l'élément d'arrêt (11) peuvent être activés ou désactivés par l'unité à microprocesseur (8).
